# EUROPEAN PATENT APPLICATION

(11) **EP 4 711 450 A1**
(43) Date of publication of application: **18.03.2026**
(21) Application number: 24803515.6
(22) Date of filing: 09.05.2024
(51) Int. Cl.: C12N 9/02, A23L 5/00, C12P 7/46, C12P 7/6409

(54) **ENZYME AGENT FOR CARBOXY GROUP GENERATION AND APPLICATION THEREOF**

(30) Priority: 10.05.2023 JP 2023077788
(71) Applicant: Amano Enzyme Inc., Nagoya-shi Aichi 460-8630 (JP)
(72) Inventor: MATSUDA Kanki, Kakamigahara-shi, Gifu 509-0109 (JP); KODAMA Yusaku, Kakamigahara-shi, Gifu 509-0109 (JP)
(74) Representative: Witthoff Jaekel Steinecke Patentanwälte PartG mbB
(86) International application number: PCT/JP2024/017215
(87) International publication number: WO 2024/232406

(57) **Abstract**

It is an object of the present invention to provide a novel enzyme having a carboxy group-generating activity. The present invention relates to a carboxy group-generating enzyme agent, containing a polypeptide described in any of the following (1) to (3):
(1) a polypeptide consisting of the amino acid sequence as set forth in any of the amino acid sequence as set forth in any of SEQ ID NOs: 1, 2, 4, 5, 7, 8, 10 and 11;
(2) a polypeptide having a substitution, an addition, an insertion and/or a deletion of one or several amino acid residues in the amino acid sequence as set forth in any of SEQ ID NOs: 1, 2, 4, 5, 7, 8, 10 and 11, and having a carboxy group-generating activity; and
(3) a polypeptide having a sequence identity of 90% or more to the amino acid sequence as set forth in any of SEQ ID NOs: 1, 2, 4, 5, 7, 8, 10 and 11, and having a carboxy group-generating activity.

## Description

### Technical Field

The present invention relates to a novel carboxy group-generating enzyme agent. In addition, the present invention relates to: a method for producing a polyvalent carboxylic acid-containing composition, using a carboxy group-generating enzyme agent; a method for carboxylating fatty acid; and a method for producing a polyvalent carboxylic acid-containing food product.

### Background Art

Carboxylic acid is used as a main raw materials for chemical products. Among others, dicarboxylic acid is used as a raw material for polymers such as polyamide and polyester, plasticizers, lubricants, fragrances, etc. For example, sebacic acid, which is one type of dicarboxylic acid, is utilized as a raw material for 6,10-nylon. In addition, brassylic acid is also used as a raw material for ethylene brassylate, a musk-based fragrance.

As methods for producing dicarboxylic acid, chemical synthesis using petroleum raw materials and microbial fermentation have been known. For example, Patent Document 1 discloses a method for producing long-chain dicarboxylic acid, which includes culturing microorganisms in a medium to which fatty acid ester as a substrate and water-soluble alcohol as a carbon source are added. However, chemical synthesis using petroleum raw materials has been problematic in terms of environmental burden, and production of dicarboxylic acid utilizing microbial fermentation has been problematic in that it is difficult to control microorganisms and in terms of contamination with metabolites derived from microorganisms. Thus, a simpler method with less environmental burden has been desired.

As a method for producing dicarboxylic acid, a production method of allowing an enzyme to act on fatty acid has also been known. For example, Patent Document 2 and Non-Patent Document 1 report that dicarboxylic acids such as azelaic acid are obtained by allowing laccase to act on fatty acid in the presence of a mediator such as 1-hydroxybenzotriazole. Moreover, Non-Patent Document 2 reports that the dicarboxylic acid, hexadecanedioic acid, is obtained by allowing cytochrome P450 52A3 to act on hexadecane.

Furthermore, Non-Patent Document 3 reports that dicarboxylic acids such as azelaic acid and brassylic acid are obtained by allowing peroxygenase (MroUPO) produced by *Marasmius rotula* to act on decanoic acid or myristic acid. Further, Non-Patent Document 4 reports that dicarboxylic acids that become smaller by two carbon atoms are obtained by allowing peroxygenase (AaeUPO) produced by *Agrocybe aegerita* or peroxygenase (rCciUPO) derived from *Coprinopsis cinerea* to act on stearic acid or fatty acids such as oleic acid, linoleic acid, and linolenic acid.

### Prior Art Documents

### Patent Documents

Patent Document 1: JP Patent Publication No. 2014-168440 A
Patent Document 2: JP Patent Publication No. 2006-262728 A

### Non-Patent Documents

Non-Patent Document 1: Bioscience Biotechnology and Biochemistry, 2016 Vol. 80, No. 11, 2132-2137
Non-Patent Document 2: THE JOURNAL OF BIOLOGICAL CHEMISTRY Vol. 273, No. 49, Issue of December 4, pp. 32528-32534, 1998
Non-Patent Document 3: Chemistry a European Journal 2017, 23, 1-6
Non-Patent Document 4: Antioxidants 2022, 11, 744

### Summary of Invention

### Objects to be Solved by the Invention

As described above, carboxylic acids and dicarboxylic acids are expected to be utilized in a wide range of applications. There have been various reports regarding enzymatic production of dicarboxylic acids. However, for example, the method using cytochrome P450 requires NADPH as a cofactor, and the method using a laccase catalyst requires a mediator such as 1-hydroxybenzotriazole, which leads to problems such as increased costs. In addition, peroxygenase derived from basidiomycetes is inexpensive because it uses peroxide as a cofactor, but it has not yet been put to practical use, and it has been further desired to develop enzymes.

Thus, it is an object of the present invention to provide a novel enzyme having a carboxy group-generating activity.

### Means for Solving the Objects

Examples of specific aspects of the present invention will be described below.

[1] A carboxy group-generating enzyme agent, containing a polypeptide described in any of the following (1) to (3):
   (1) a polypeptide consisting of the amino acid sequence as set forth in any of the amino acid sequence as set forth in any of SEQ ID NOs: 1, 2, 4, 5, 7, 8, 10 and 11;
   (2) a polypeptide having a substitution, an addition, an insertion and/or a deletion of one or several amino acid residues in the amino acid sequence as set forth in any of SEQ ID NOs: 1, 2, 4, 5, 7, 8, 10 and 11, and having a carboxy group-generating activity; and
   (3) a polypeptide having a sequence identity of 90% or more to the amino acid sequence as set forth in any of SEQ ID NOs: 1, 2, 4, 5, 7, 8, 10 and 11, and having a carboxy group-generating activity.
[2] The carboxy group-generating enzyme agent according to [1], which is for use in dicarboxylic acid generation.
[3] A method for producing a polyvalent carboxylic acid-containing composition, including allowing the polypeptide or the carboxy group-generating enzyme agent according to [1] or [2] to act on a fatty acid-containing composition.
[4] The method for producing a polyvalent carboxylic acid-containing composition according to [3], in which the fatty acid-containing composition contains decanoic acid or myristic acid.
[5] The method for producing a polyvalent carboxylic acid-containing composition according to [3] or [4], in which the polyvalent carboxylic acid-containing composition is a dicarboxylic acid-containing composition.
[6] A method for carboxylating fatty acid, including allowing the polypeptide or the carboxy group-generating enzyme agent according to [1] or [2] to act on fatty acid.
[7] A method for producing a polyvalent carboxylic acid-containing food product, including allowing the polypeptide or the carboxy group-generating enzyme agent according to claim [1] or [2] to act on a fatty acid-containing food product.
[8] The method for producing a polyvalent carboxylic acid-containing food product according to [7], in which the polyvalent carboxylic acid-containing food product is a dicarboxylic acid-containing food product.

Moreover, the present invention has the following configurations.
[A] A method for generating polyvalent carboxylic acid, including allowing the polypeptide described in any of the above (1) to (3) to act on fatty acid.
[B] An enzyme agent containing the polypeptide described in any of the above (1) to (3), which is for use in carboxylation of fatty acid.
[C] Use of an enzyme agent containing the polypeptide described in any of the above (1) to (3), which is for use in carboxylation of fatty acid.
[D] Use of an enzyme agent containing the polypeptide described in any of the above (1) to (3), which is for use in the improvement of food products.
[E] A polyvalent carboxylic acid-containing composition, which is produced by the method for producing a polyvalent carboxylic acid-containing composition according to any one of the above [3] to [5].
[F] A polyvalent carboxylic acid-containing food product, which is produced by the method for producing a polyvalent carboxylic acid-containing food product according to the above [7] or [8].
[G] A fatty acid-shortening enzyme agent, containing the polypeptide described in any of the above (1) to (3).
[H] A hydroxyl group-generating enzyme agent, containing the polypeptide described in any of the above (1) to (3).
[I] A method for shortening fatty acid, including allowing a fatty acid-shortening enzyme agent that contains the polypeptide described in any of the above (1) to (3), to act on fatty acid.
[J] A hydroxylation method or a carboxylation method, including allowing a carboxy group-generating enzyme agent (or hydroxyl group-generating enzyme agent) that contains the polypeptide described in any of the above (1) to (3), to act on an alkane-containing composition or a fatty acid ester-containing composition.

### Advantageous Effects of Invention

According to the present invention, a novel enzyme agent having a carboxy group-generating activity can be provided.

### Brief Description of Drawings

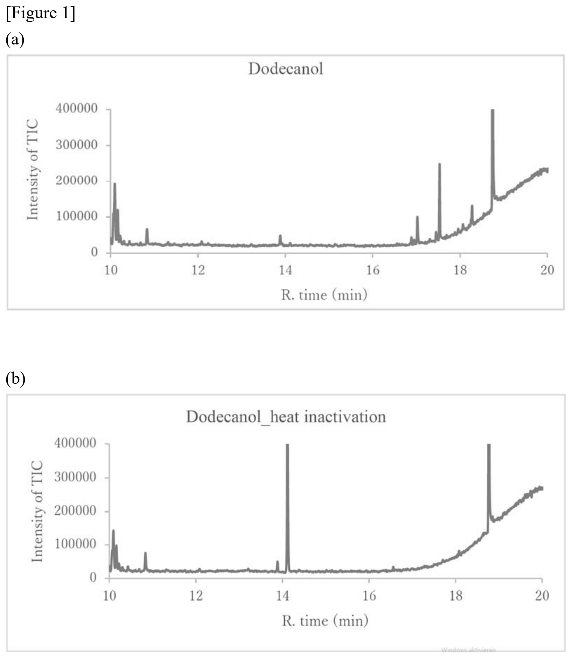

[Figure 1] Figure 1 shows the results of chromatography when dodecanol was used as a substrate in Test Example 2.

### Embodiments of Carrying out the Invention

The present invention will be described in detail below. The constitutive elements of the present invention given below may be described based on some typical embodiments or specific examples. However, the present invention should not be limited to such embodiments. In the present description, the numerical range expressed with the wording "a number to another number" means the range that falls between the former number indicating the lower limit value of the range and the latter number indicating the upper limit value thereof.

In the present description, the 20 types of amino acid residues in the amino acid sequences may be expressed by one-letter abbreviations in some cases. In that case, glycine (Gly) is G, alanine (Ala) is A, valine (Val) is V, leucine (Leu) is L, isoleucine (Ile) is I, phenylalanine (Phe) is F, tyrosine (Tyr) is Y, tryptophan (Trp) is W, serine (Ser) is S, threonine (Thr) is T, cysteine (Cys) is C, methionine (Met) is M, Aspartic acid (Asp) is D, glutamic acid (Glu) is E, asparagine (Asn) is N, glutamine (Gln) is Q, lysine (Lys) is K, arginine (Arg) is R, histidine (His) is H, and proline (Pro) is P. Moreover, in the present description, the left end of the amino acid sequences displayed is the N-terminus, and the right end thereof is the C-terminus.

In the present description, "non-polar amino acids" include alanine, valine, leucine, isoleucine, proline, methionine, phenylalanine and tryptophan. "Uncharged amino acids" include glycine, serine, threonine, cysteine, tyrosine, asparagine and glutamine. "Acidic amino acids" include aspartic acid and glutamic acid. "Basic amino acids" include lysine, arginine and histidine.

In the present description, "substitution" refers not only to the case where amino acid residue substitution is artificially introduced, but also to the case where amino acid residue substitution is introduced naturally, that is, the case where the amino acid residue was originally different. In the present description, the substitution of amino acid residues may be an artificial substitution, or a natural substitution, but an artificial substitution is preferable.

### (Carboxy group-generating enzyme agent)

The present embodiment relates to a carboxy group-generating enzyme agent, containing a polypeptide described in any of the following (1) to (3):
(1) a polypeptide consisting of the amino acid sequence as set forth in any of the amino acid sequence as set forth in any of SEQ ID NOs: 1, 2, 4, 5, 7, 8, 10 and 11;
(2) a polypeptide having a substitution, an addition, an insertion and/or a deletion of one or several amino acid residues in the amino acid sequence as set forth in any of SEQ ID NOs: 1, 2, 4, 5, 7, 8, 10 and 11, and having a carboxy group-generating activity; and
(3) a polypeptide having a sequence identity of 90% or more to the amino acid sequence as set forth in any of SEQ ID NOs: 1, 2, 4, 5, 7, 8, 10 and 11, and having a carboxy group-generating activity.

The carboxy group-generating enzyme agent of the present embodiment contains, as an active ingredient, the polypeptide described in any of the above (1) to (3). It is to be noted that the polypeptide described in any of the above (1) to (3) is a carboxy group-generating enzyme, and that the carboxy group-generating enzyme agent may consist of the aforementioned carboxy group-generating enzyme.

In the present embodiment, the carboxy group-generating enzyme agent is preferably for use in generation of dicarboxylic acid. In the present description, the dicarboxylic acid refers to a dicarboxylic acid having carboxy groups at both termini of the carbon chain, and examples thereof may include malonic acid (C3), malic acid (C4), fumaric acid (C4), succinic acid (C4), itaconic acid (C5), glutaric acid (C5), adipic acid (C6), pimelic acid (C7), suberic acid (C8), azelaic acid (C9), sebacic acid (decanedioic acid) (C10), undecanedioic acid (C11), dodecanedioic acid (C12), tridecanedioic acid (brassylic acid) (C13), and tetradecanedioic acid (C14). The long-chain dicarboxylic acid is a dicarboxylic acid containing 10 or more carbon atoms.

It is to be noted that, in the present description, when Cx is used to express fatty acids or hydrocarbons, it means that such fatty acid or hydrocarbon contains an x number of carbon atoms. For example, malonic acid (C3) means that it is fatty acid containing 3 carbon atoms.

The carboxy group-generating enzyme agent of the present embodiment contains the polypeptide described in any of the above (1) to (3), and it is a novel enzyme agent having a carboxy group-generating activity. Since carboxylic acid or dicarboxylic acid is expected to be utilized in a wide range of applications as a basic raw material for chemical products, the novel enzyme agent of the present embodiment is highly useful as a new enzyme that generates carboxylic acid or dicarboxylic acid. In addition, by using the novel enzyme agent of the present embodiment, it is expected that a method for generating carboxylic acid or dicarboxylic acid at low costs with high efficiency will be established.

The content of the polypeptide (carboxy group-generating enzyme) described in any of the above (1) to (3) in the carboxy group-generating enzyme agent of the present embodiment is not particularly limited, and it can be set, as appropriate, within a range in which appropriate catalytic activity is exhibited, for example, when dicarboxylic acid is generated using fatty acid as a substrate. The content of the polypeptide (carboxy group-generating enzyme) in the carboxy group-generating enzyme agent is different depending on the type, and it may be, for example, 0.001% to 50% by mass, or may be 0.01% to 20% by mass, or may also be 0.1% to 10% by mass, with respect to the total mass of the carboxy group-generating enzyme agent.

The carboxy group-generating enzyme used in the present embodiment is an enzyme that has heme b as a cofactor. Heme b exhibits absorbance having a characteristic wavelength when it binds to pyridine under alkaline conditions. The concentration of heme b is determined from the molar extinction coefficient (34.7 mM⁻¹ cm⁻¹) at this wavelength (557 nm). The enzyme concentration can be calculated, assuming that the concentration of heme b is the enzyme concentration.

The activity of the carboxy group-generating enzyme can be determined from the change in absorbance at 469 nm when a 5 mM 2,6-dimethoxyphenol solution (0.1 M potassium phosphate/0.05 M potassium acetate buffer, pH 7.0, 0.5 mM hydrogen peroxide) is used as a substrate and is treated at 40°C. In the present description, the amount of an enzyme that oxidizes 1 nmol of substrate per minute under the above-described conditions is defined as 1 unit (1 U).

The carboxy group-generating enzyme agent of the present embodiment may consist of only a carboxy group-generating enzyme, or may contain hydrogen peroxide in addition to a carboxy group-generating enzyme. When a carboxy group-generating enzyme agent contains hydrogen peroxide, generation of carboxy groups, for example, generation of dicarboxylic acids, is promoted.

In addition, the carboxy group-generating enzyme agent of the present embodiment may contain other components in addition to the carboxy group-generating enzyme, to such an extent that such other components do not affect the effects of the present invention. Examples of other components may include other enzymes other than the carboxy group-generating enzyme, additives, and culture residues generated in the step of preparing the carboxy group-generating enzyme, etc.

Examples of other enzymes other than the carboxy group-generating enzyme may include amylase (α-amylase, β-amylase, and glucoamylase), glucosidase (α-glucosidase and β-glucosidase), galactosidase (α-galactosidase and β-galactosidase), protease (acid protease, neutral protease, and alkaline protease), peptidase (leucine peptidase and aminopeptidase), lipase, esterase, cellulase, phosphatase (acid phosphatase and alkaline phosphatase), nuclease, deaminase, oxidase, dehydrogenase, glutaminase, pectinase, dextranase, transglutaminase, protein deamidase, pullulanase, and peroxidase. These other enzymes may be one type or multiple types.

Examples of the additives that may be contained in the carboxy group-generating enzyme agent may include an excipient, a buffer agent, a suspending agent, a stabilizer, a preservative, an antiseptic, and a normal saline. Examples of the excipient may include starch, dextrin, maltose, trehalose, lactose, D-glucose, sorbitol, D-mannitol, white sugar, and glycerol. Examples of the buffer agent may include phosphate, citrate, and acetate. Examples of the stabilizer may include propylene glycol and ascorbic acid. Examples of the preservative may include phenol, benzalkonium chloride, benzyl alcohol, chlorobutanol, and methylparaben. Examples of the antiseptic may include ethanol, benzalkonium chloride, paraoxybenzoic acid, and chlorobutanol. These additives may be one type or multiple types.

The form of the carboxy group-generating enzyme agent of the present embodiment is not particularly limited, and examples thereof may include a powder form, a solid form, a gel form, a liquid form (slurry), and a form immobilized on a carrier.

### (Polypeptide (carboxy group-generating enzyme))

The carboxy group-generating enzyme agent of the present embodiment contains a polypeptide described in any of the following (1) to (3). The polypeptide described in any of the following (1) to (3) has a carboxy group-generating activity, and is a carboxy group-generating enzyme.
(1) A polypeptide consisting of the amino acid sequence as set forth in any of the amino acid sequence as set forth in any of SEQ ID NOs: 1, 2, 4, 5, 7, 8, 10 and 11.
(2) A polypeptide having a substitution, an addition, an insertion and/or a deletion of one or several amino acid residues in the amino acid sequence as set forth in any of SEQ ID NOs: 1, 2, 4, 5, 7, 8, 10 and 11, and having a carboxy group-generating activity.
(3) A polypeptide having a sequence identity of 90% or more to the amino acid sequence as set forth in any of SEQ ID NOs: 1, 2, 4, 5, 7, 8, 10 and 11, and having a carboxy group-generating activity.

The type of the carboxy group-generating enzyme is not particularly limited. The carboxy group-generating enzyme may be, for example, a carboxy group-generating enzyme derived from basidiomycetes. Preferred examples of the carboxy group-generating enzyme may include a carboxy group-generating enzyme derived from genus Marasmius, a carboxy group-generating enzyme derived from genus Dendrothele, a carboxy group-generating enzyme derived from genus Mycena, and a carboxy group-generating enzyme derived from genus Armillaria. More preferred examples of the carboxy group-generating enzyme may include a carboxy group-generating enzyme derived from *Marasmius fiardii*, a carboxy group-generating enzyme derived from *Dendrothele bispora*, a carboxy group-generating enzyme derived from *Mycena venus,* and a carboxy group-generating enzyme derived from *Armillaria ostoyae.* In addition, the carboxy group-generating enzyme agent may contain one or two or more types of carboxy group-generating enzymes. Moreover, the carboxy group-generating enzyme agent may be one agent or two or more agents.

Among others, it is preferable that the polypeptide consisting of the amino acid sequence as set forth in SEQ ID NO: 1 is a carboxy group-generating enzyme derived from *Marasmius fiardii,* that the polypeptide consisting of the amino acid sequence as set forth in SEQ ID NO: 4 is a carboxy group-generating enzyme derived from *Dendrothele bispora*, that the polypeptide consisting of the amino acid sequence as set forth in SEQ ID NO: 7 is a carboxy group-generating enzyme derived from *Mycena venus,* and that the polypeptide consisting of the amino acid sequence as set forth in SEQ ID NO: 10 is a carboxy group-generating enzyme derived from *Armillaria ostoyae.*

The amino acid sequence as set forth in SEQ ID NO: 2 is the amino acid sequence as set forth in SEQ ID NO: 1, onto the C-terminal side of which a polyhistidine tag has been added; the amino acid sequence as set forth in SEQ ID NO: 5 is the amino acid sequence as set forth in SEQ ID NO: 4, onto the C-terminal side of which a polyhistidine tag has been added; the amino acid sequence as set forth in SEQ ID NO: 8 is the amino acid sequence as set forth in SEQ ID NO: 7, onto the C-terminal side of which a polyhistidine tag has been added; and the amino acid sequence as set forth in SEQ ID NO: 11 is the amino acid sequence as set forth in SEQ ID NO: 10, onto the C-terminal side of which a polyhistidine tag has been added. In addition, the amino acid sequence as set forth in SEQ ID NO: 3 is the amino acid sequence as set forth in SEQ ID NO: 1, onto the C-terminal side of which a polyhistidine tag has been added, and onto the N-terminal side of which a signal sequence has been added; the amino acid sequence as set forth in SEQ ID NO: 6 is the amino acid sequence as set forth in SEQ ID NO: 4, onto the C-terminal side of which a polyhistidine tag has been added, and onto the N-terminal side of which a signal sequence has been added; the amino acid sequence as set forth in SEQ ID NO: 9 is the amino acid sequence as set forth in SEQ ID NO: 7, onto the C-terminal side of which a polyhistidine tag has been added, and onto the N-terminal side of which a signal sequence has been added; and the amino acid sequence as set forth in SEQ ID NO: 12 is the amino acid sequence as set forth in SEQ ID NO: 10, onto the C-terminal side of which a polyhistidine tag has been added, and onto the N-terminal side of which a signal sequence has been added. The carboxy group-generating enzyme agent of the present embodiment may contain the polypeptide consisting of the amino acid sequence as set forth in any of SEQ ID Nos: 1 to 12.

The polypeptide of the above (2) is a polypeptide having a substitution, an addition, an insertion and/or a deletion of one or several amino acid residues in the amino acid sequence as set forth in any of SEQ ID NOs: 1, 2, 4, 5, 7, 8, 10 and 11, and having a carboxy group-generating activity. Modification of amino acid(s) may be inserted into any given difference site(s). In addition, modification of amino acid(s) may be one type of modification (e.g., only substitution) selected from substitution, addition, insertion and deletion, or may also be two or more types of modifications (e.g., a substitution and insertion). In the polypeptide of the above (2), the number of differences in amino acids in any given difference sites may be one or several. The number of differences is, for example, 1 to 80, 1 to 70, 1 to 60, 1 to 50, 1 to 40, 1 to 30, 1 to 20, 1 to 10, 1 to 8, 1 to 7, 1 to 6, 1 to 5, 1 to 4, 1 to 3, or 1 or 2. Besides, since the amino acid sequence as set forth in SEQ ID NO: 3 is the amino acid sequence as set forth in SEQ ID NO: 2, to which a signal sequence has been added, it corresponds to a polypeptide having an addition of several amino acid residues, as described in (2) above. Likewise, SEQ ID Nos: 6, 9 and 12 correspond to polypeptides having an addition of several amino acid residues, respectively.

The polypeptide of the above (3) has a sequence identity of preferably 60% or more, more preferably 70% or more, even more preferably 80% or more, further preferably 90% or more, still further preferably 95% or more, particularly preferably 97% or more, and most preferably 99% or more, with respect to the amino acid sequence as set forth in any of SEQ ID NOs: 1, 2, 4, 5, 7, 8, 10 and 11. In addition, the polypeptide of the above (3) also has a carboxy group-generating activity.

In the polypeptide of the above (3), the sequence identity to the amino acid sequence as set forth in any of SEQ ID NOs: 1, 2, 4, 5, 7, 8, 10 and 11 means a sequence identity calculated by comparing the concerned polypeptide with the amino acid sequence as set forth in any of SEQ ID NOs: 1, 2, 4, 5, 7, 8, 10 and 11. In the present description, the term "sequence identity" refers to the identity value of the amino acid sequence, which is obtained by the bl2seq program (Tatiana A. Tatsusova, Thomas L. Madden, FEMS Microbiol. Lett., Vol. 174, pp. 247-250, 1999) of BLASTPACKAGE [sgi32 bit edition, Version 2.0.12; available from National Center for Biotechnology Information (NCBI)]. The sequence identity is calculated by setting parameters to Gap insertion Cost value: 11, Gap extension Cost value: 1.

When an amino acid substitution is introduced into any given difference sites of the polypeptides of the above (2) and (3), one preferred aspect of the amino acid substitution to be introduced may be a conservative substitution. Examples of the amino acid substitution in the polypeptides of the above (2) and (3) may include: a substitution with another nonpolar amino acid if the amino acid before substitution is a nonpolar amino acid; a substitution with another uncharged amino acid if the amino acid before substitution is an uncharged amino acid; a substitution with another acidic amino acid if the amino acid before substitution is an acidic amino acid; and a substitution with another basic amino acid if the amino acid before substitution is a basic amino acid.

Besides, in the polypeptides of the above (2) and (3), since the amino acid residues corresponding to: the positions 23 (cysteine residue), 94 (histidine residue), and 165 (aspartic acid residue) in the amino acid sequence as set forth in SEQ ID No: 1; the positions 26 (cysteine residue), 95 (histidine residue), and 166 (aspartic acid residue) in the amino acid sequence as set forth in SEQ ID NO: 4; the positions 20 (cysteine residue), 89 (histidine residue), and 161 (aspartic acid residue) in the amino acid sequence as set forth in SEQ ID NO: 7; and the positions 21 (cysteine residue), 90 (histidine residue), and 161 (aspartic acid residue) in the amino acid sequence as set forth in SEQ ID NO: 10 are considered to be active catalyst residues. Accordingly, it is desirable not to introduce substitution or deletion into these sites. Besides, in the present description, in the polypeptides of the above (2) and (3), amino acid residues other than the amino acid residues corresponding to the aforementioned active catalyst residues are also referred to as "any given difference sites" at times. In the present description, the "any given difference sites" are sites in which differences are permitted as long as they do not significantly affect the properties of the polypeptide. A polypeptide that has a difference in the amino acid sequence at any given difference site but has a carboxy group-generating activity equivalent to or higher than that of the polypeptide of the above (1) is referred to as a different body of the polypeptide of the above (1). In other words, the polypeptides the above (2) and (3) are different bodies of the polypeptide of the above (1).

The polypeptides of the above (1) to (3) may be a part of a larger protein (for example, a fusion protein). Examples of a sequence to be added to such a fusion protein may include sequences that are useful for purification, such as multiple histidine residues, and additional sequences that ensure stability during recombinant production.

### (DNA)

The nucleotide sequences encoding the polypeptides consisting of the amino acid sequences as set forth in SEQ ID Nos: 1 to 12 are shown in SEQ ID NOs: 13 to 24, respectively. The nucleotide sequences encoding the polypeptides consisting of the amino acid sequences as set forth in any of SEQ ID NOs: 1 to 12 include sequences equivalent to the nucleotide sequences as set forth in SEQ ID NOs: 13 to 24.

The sequences equivalent to the nucleotide sequences as set forth in SEQ ID NOs: 13 to 24 gas a homology of preferably 75% or more, more preferably 80% or more, even more preferably 85% or more, further preferably 90% or more, still further preferably 95% or more, and particularly preferably 99% or more, to the nucleotide sequences as set forth in SEQ ID NOs: 13 to 24. Even in the case of DNA having a sequence different from the nucleotide sequence as set forth in any of SEQ ID NOs: 13 to 24, if the DNA encodes a polypeptide having a carboxy group-generating activity, it can be said that the DNA is equivalent to the nucleotide sequence as set forth in any of SEQ ID NOs: 13 to 24.

The "homology" of DNA is calculated using publicly disclosed or commercially available software having an algorithm of comparing the sequence with a reference sequence used as a query sequence. Specifically, BLAST, FASTA, GENETYX (manufactured by Fukuoka Software Development Co., Ltd.) or the like can be used, and these may be used with the default parameters set.

DNAs having the nucleotide sequences as set forth in SEQ ID NOs: 13 to 24 or DNAs equivalent thereto can also be isolated from microorganisms that produce the aforementioned polypeptides. For example, using the genomic DNA of *Marasmius fiardii* PR as a template, DNA of interest can be isolated from the genome of the above-mentioned microorganism by performing PCR or a hybridization method using primers or probes designed from known amino acid sequence information taking gene degeneracy into consideration, or using primers or probes designed based on known nucleotide sequence information.

The DNAs having the nucleotide sequences as set forth in SEQ ID NOs: 13 to 24 or the DNAs equivalent thereto may be artificially synthetized genes. In this case, for example, an artificially synthesized gene can be produced, in which the codons are optimized based on the codon frequency of *Aspergillus oryzae.* The DNA includes many types of DNAs derived from codon degeneracy. Many types of DNA encoding an identical amino acid sequence can be easily, artificially produced using known genetic engineering techniques. For example, in the production of a protein by genetic engineering, if the codon used on the original gene encoding the protein of interest is one that is used at low frequency in the host, the amount of the protein produced may be low. In such a case, the codon usage frequency can be optimized for the host without changing the encoded amino acid sequence, thereby achieving an increase in the amount of the protein of interest produced.

As an index of codon usage frequency, the total of the host optimal codon usage frequency of each codon may be adopted. The optimal codon is defined to be a codon with the highest usage frequency among the codons corresponding to an identical amino acid. The codon usage frequency is not particularly limited, as long as it is optimized for the host. For example, the following are examples of optimal codons for *E. coli.*

F: phenylalanine (ttt), L: leucine (ctg), I: isoleucine (att), M: methionine (atg), V: valine (gtg), Y: tyrosine (tat), stop codon (taa), H: histidine (cat), Q: glutamine (cag), N: asparagine (aat), K: lysine (aaa), D: aspartic acid (gat), E: glutamic acid (gaa), S: serine (age), P: proline (ccg), T: threonine (acc), A: alanine (gcg), C: cysteine (tgc), W: tryptophan (tgg), R: arginine (cgc), and G: glycine (ggc).

Examples of the method of introducing a mutation into a gene to artificially modify an amino acid sequence may include known methods such as a Kunkel method and a Gapped Duplex method, and the use of mutation introduction kits utilizing a site-specific mutagenesis method, such as QuikChange^{™} Site-Directed Mutagenesis Kit (Stratagene), GeneTailor^{™} Site-Directed Mutagenesis System (Invitrogen Co., Ltd.), and TaKaRa Site-Directed Mutagenesis System (Mutan-K, Mutan-Super Express Km, etc.: Takara Bio, Inc.).

The nucleotide sequence of DNA can be confirmed by sequencing using a common method. For example, the nucleotide sequence of DNA can be confirmed by a dideoxynucleotide chain termination method (Sanger et al. (1977) Proc. Natl. Acad. Sci. USA 74: 5463), etc. In addition, the sequence can be analyzed utilizing an appropriate DNA Sequencer.

Whether or not the obtained DNA is DNA encoding a polypeptide of interest can be confirmed by comparing the determined nucleotide sequence with the nucleotide sequences as set forth in SEQ ID NOs: 13 to 24. Otherwise, such confirmation can also be carried out by measuring the catalytic activity of the expressed polypeptide (i.e., carboxy group-generating activity).

### (Method for producing polyvalent carboxylic acid-containing composition/method for producing polyvalent carboxylic acid-containing food product)

The present embodiment relates to a method for producing a polyvalent carboxylic acid-containing composition, including allowing the polypeptide described in any of the above (1) to (3) or the aforementioned carboxy group-generating enzyme agent to act on a fatty acid-containing composition. In addition, the present embodiment relates to a method for producing a polyvalent carboxylic acid-containing food product, including allowing the polypeptide described in any of the above (1) to (3) or the aforementioned carboxy group-generating enzyme agent to act on a fatty acid-containing food product. In the present embodiment, a carboxy group-generating enzyme is allowed to act on a fatty acid-containing composition or a fatty acid-containing food product, so that a polyvalent carboxylic acid-containing composition or a polyvalent carboxylic acid-containing food product can be obtained. The polyvalent carboxylic acid-containing composition is preferably a dicarboxylic acid-containing composition, and the polyvalent carboxylic acid-containing food product is preferably a dicarboxylic acid-containing food product. In addition, the fatty acid contained in the fatty acid-containing composition or the fatty acid-containing food product is preferably long-chain fatty acid. That is to say, a more preferred aspect of the present embodiment relates to a method for producing a long-chain dicarboxylic acid-containing composition or a long-chain dicarboxylic acid-containing food product.

The present embodiment may relate to a method for producing a polyvalent carboxylic acid-containing composition, including allowing the polypeptide described in any of the above (1) to (3) or the aforementioned carboxy group-generating enzyme agent to act on an alcohol-containing composition. In addition, the present embodiment relates to a method for producing a polyvalent carboxylic acid-containing food product, including allowing the polypeptide described in any of the above (1) to (3) or the aforementioned carboxy group-generating enzyme agent to act on an alcohol-containing food product. In the present embodiment, a carboxy group-generating enzyme is allowed to act on an alcohol-containing composition or an alcohol-containing food product, so that a polyvalent carboxylic acid-containing composition or a polyvalent carboxylic acid-containing food product can be obtained. The polyvalent carboxylic acid-containing composition is preferably a dicarboxylic acid-containing composition, and the polyvalent carboxylic acid-containing food product is preferably a dicarboxylic acid-containing food product. In addition, the alcohol contained in the alcohol-containing composition or the alcohol-containing food product is preferably long-chain aliphatic alcohol. That is to say, a more preferred aspect of the present embodiment relates to a method for producing a long-chain dicarboxylic acid-containing composition or a long-chain dicarboxylic acid-containing food product.

Moreover, the carboxy group-generating enzyme of the present embodiment also catalyzes a reaction of shortening fatty acid. For this reason, the carboxy group-generating enzyme may also be a fatty acid-shortening enzyme. That is, another aspect of the present embodiment relates to a method for producing a processed fatty acid-containing composition, including allowing an enzyme agent for shortening fatty acid that contains the polypeptide described in any of the above (1) to (3), to act on a fatty acid-containing composition. For example, a fatty acid-shortening enzyme is allowed to act on a long-chain fatty acid-containing composition, so that the long-chain fatty acid having a long chain length can be shortened and so that fatty acid having a shorter carbon chain length than that of the original long-chain fatty acid can be obtained.

The carboxy group-generating enzyme of the present embodiment also catalyzes a reaction of shortening long-chain aliphatic alcohol. For this reason, the carboxy group-generating enzyme may also be a long-chain aliphatic alcohol-shortening enzyme. That is, another aspect of the present embodiment relates to a method for producing a processed alcohol-containing composition, including allowing an enzyme agent for shortening long-chain aliphatic alcohol that contains the polypeptide described in any of the above (1) to (3), to act on a long-chain aliphatic alcohol-containing composition. For example, a long-chain aliphatic alcohol-shortening enzyme is allowed to act on a long-chain aliphatic alcohol-containing composition, so that aliphatic alcohol having a long chain length can be shortened, and so that aliphatic alcohol having a shorter carbon chain length than that of the original long-chain aliphatic alcohol can be obtained.

As a further aspect, the carboxy group-generating enzyme of the present embodiment catalyzes a reaction of hydroxylating or carboxylating the terminal alkyl group of alkane or fatty acid ester to generate alcohol or carboxylic acid. For this reason, the carboxy group-generating enzyme may be a hydroxyl group-generating enzyme. It is to be noted that the terminal alkyl group of alkane or fatty acid ester is preferably carboxylated after it has been hydroxylated. That is to say, another aspect of the present embodiment relates to a method for producing an alcohol-containing composition or a carboxylic acid-containing composition, including allowing a carboxy group-generating enzyme agent (hydroxyl group-generating enzyme agent) that contains the polypeptide described in any of the above (1) to (3), to act on an alkane-containing composition or a fatty acid ester-containing composition.

### < Fatty acid-containing composition/fatty acid-containing food product >

The fatty acid-containing composition contains fatty acid. In addition, the fatty acid-containing composition may be a composition containing a compound having fatty acid as a partial structure. Herein, fatty acids are classified into unsaturated fatty acids and saturated fatty acids, and unsaturated fatty acids are fatty acids having one or more carbon double bonds. Monovalent unsaturated fatty acids are unsaturated fatty acids having only one carbon double bond. On the other hand, polyvalent unsaturated fatty acids are unsaturated fatty acids having two or more carbon double bonds, and are divided into ω6 fatty acids and ω3 fatty acids. Specifically, the monovalent unsaturated fatty acids include myristoleic acid, palmitoleic acid, oleic acid, gondoic acid, erucic acid, nervonic acid, hexacosenoic acid, and octacosenoic acid. The ω3 polyvalent unsaturated fatty acids include α-linolenic acid, stearidonic acid, eicosatetraenoic acid, eicosapentaenoic acid, and docosahexaenoic acid. The ω6 polyvalent unsaturated fatty acids include linoleic acid, γ-linolenic acid, dihomo-γ-linolenic acid, arachidonic acid, and docosapentaenoic acid. In addition, fatty acids are also classified into long-chain fatty acids, medium-chain fatty acids, and short-chain fatty acids, based on their chain length. Long-chain fatty acids are fatty acids containing 10 or more carbon atoms, and short-chain fatty acids are fatty acids containing 6 or less carbon atoms.

Among others, the fatty acid is preferably long-chain fatty acid, and the fatty acid more preferably includes at least one selected from the group consisting of decanoic acid (capric acid), myristic acid, lauric acid, palmitic acid, palmitoleic acid, stearic acid and oleic acid, and the fatty acid is further preferably decanoic acid or myristic acid. It is to be noted that the fatty acid contained in the fatty acid-containing composition may be of a single type alone or may also be multiple types.

When the composition containing a compound having fatty acid as a partial structure is used as a fatty acid-containing composition, the compound having fatty acid as a partial structure is preferably fatty acid ester, and is more preferably glyceride. That is to say, the composition containing a compound having fatty acid as a partial structure is preferably a fatty acid ester-containing composition, and is more preferably a glyceride-containing composition. The glyceride-containing composition may be, for example, oil and fat. The glyceride-containing composition contains glyceride, and as such glyceride, triglyceride, diglyceride and monoglyceride can be used. It is to be noted that the glyceride is formed by binding at least one fatty acid to glycerin.

In the present embodiment, the fatty acid-containing composition may contain other optional components in addition to the fatty acid, or may also consist of the fatty acid or a compound having the fatty acid as a partial structure. Examples of such other optional components may include glycerin, salts (buffer salts), and hydrogen peroxide.

The fatty acid-containing composition may be a fatty acid-containing food product. The fatty acid-containing food product is a food product or a food material that contains the aforementioned fatty acid. Examples of such a food product or a food material may include edible oils (olive oil, safflower oil, sunflower oil, rapeseed oil, peanut oil, rice bran oil, etc.), edible meat (beef, chicken, pork, etc.), dairy products (cheese, processed milk, nut milk, and yogurt), nuts (macadamia nuts, almonds, etc.), peanuts, sunflower seeds, eggs, egg products, olives, and avocados

When the fatty acid-containing composition is a fatty acid-containing food product, the present embodiment relates to a method for producing a polyvalent carboxylic acid-containing food product, including allowing the polypeptide described in any of the above (1) to (3) or the aforementioned carboxy group-generating enzyme agent to act on a fatty acid-containing food product. The fatty acid contained in the fatty acid-containing food product may be the aforementioned fatty acid, and the preferred range thereof is also the same. Moreover, the polyvalent carboxylic acid-containing food product is preferably a dicarboxylic acid-containing food product.

Furthermore, the fatty acid-containing composition may contain, as other optional components, a pH adjuster, an antiseptic, a stabilizer, a thickener, an antioxidant, a surfactant, an emulsifier, a solubilizer, a dissolution aid, a bulking agent, a suspending agent, salts (buffer salts), hydrogen peroxide, and the like. Examples of the pH adjuster may include an acetate buffer, a phosphate buffer, an MES buffer, a HEPES buffer, a PIPES buffer, a Bis-tris buffer, and a MOPS buffer.

The content of the fatty acid contained in the fatty acid-containing composition or the fatty acid-containing food product is preferably 0.001% by mass or more, more preferably 0.002% by mass or more, even more preferably 0.01% by mass or more, further preferably 0.1% by mass or more, even further preferably 1% by mass or more, still further preferably 5% by mass or more, and particularly preferably 10% by mass or more, with respect to the total mass of the fatty acid-containing composition or the fatty acid-containing food product. On the other hand, the content of the fatty acid may be 100% by mass, 90% by mass or less, 80% by mass or less, 70% by mass or less, 60% by mass or less, or 50% by mass or less, with respect to the total mass of the fatty acid-containing composition or the fatty acid-containing food product.

The form of the fatty acid-containing composition or the fatty acid-containing food product is not particularly limited as long as the enzyme reacts therewith, and it may be in any form, such as powders, a solid, gel, or a liquid (slurry). Of these, the fatty acid-containing composition is preferably in a liquid (slurry) form.

### < Alcohol-containing composition/alcohol-containing food product >

The alcohol-containing composition contains alcohol. In addition, the alcohol-containing composition may be a composition containing a compound having alcohol as a partial structure.

Among others, the alcohol is preferably long-chain aliphatic alcohol, and the long-chain aliphatic alcohol is more preferably polyhydric alcohol. The long-chain aliphatic alcohol may be, for example, aliphatic alcohol containing 8 to 40 carbon atoms, and the number of carbon atoms may be an even number or an odd number. More specific examples may include octanol, nonanol, decanol, undecanol, dodecanol, lauryl alcohol, myristyl alcohol, cetyl alcohol, oleyl alcohol, stearyl alcohol, and behenyl alcohol. It is to be noted that the alcohol contained in the alcohol-containing composition may be of a single type alone or may also be multiple types.

In the present embodiment, the alcohol-containing composition may contain other optional components in addition to the alcohol, or may also consist of the alcohol or a compound having the alcohol as a partial structure. Examples of such other optional components may include glycerin, salts (buffer salts), and hydrogen peroxide.

The alcohol-containing composition may be an alcohol-containing food product. The alcohol-containing food product is a food product or a food material that contains the aforementioned alcohol.

When the alcohol-containing composition is an alcohol-containing food product, the present embodiment relates to a method for producing a polyvalent carboxylic acid-containing food product, including allowing the polypeptide described in any of the above (1) to (3) or the aforementioned carboxy group-generating enzyme agent to act on an alcohol-containing food product. The alcohol contained in the alcohol-containing food product may be the aforementioned alcohol, and the preferred range thereof is also the same. Moreover, the polyvalent carboxylic acid-containing food product is preferably a dicarboxylic acid-containing food product.

Furthermore, the alcohol-containing composition may contain, as other optional components, a pH adjuster, an antiseptic, a stabilizer, a thickener, an antioxidant, a surfactant, an emulsifier, a solubilizer, a dissolution aid, a bulking agent, a suspending agent, salts (buffer salts), hydrogen peroxide, and the like. Examples of the pH adjuster may include an acetate buffer, a phosphate buffer, an MES buffer, a HEPES buffer, a PIPES buffer, a Bis-tris buffer, and a MOPS buffer.

The content of the alcohol contained in the alcohol-containing composition or the alcohol-containing food product is preferably 0.001% by mass or more, more preferably 0.002% by mass or more, even more preferably 0.01% by mass or more, further preferably 0.1% by mass or more, even further preferably 1% by mass or more, still further preferably 5% by mass or more, and particularly preferably 10% by mass or more, with respect to the total mass of the alcohol-containing composition or the alcohol-containing food product. On the other hand, the content of the alcohol may be 100% by mass, 90% by mass or less, 80% by mass or less, 70% by mass or less, 60% by mass or less, or 50% by mass or less, with respect to the total mass of the alcohol-containing composition or the alcohol-containing food product.

The form of the alcohol-containing composition or the alcohol-containing food product is not particularly limited as long as the enzyme reacts therewith, and it may be in any form, such as powders, a solid, gel, or a liquid (slurry). Of these, the alcohol-containing composition is preferably in a liquid (slurry) form.

### < Conditions for producing polyvalent carboxylic acid-containing composition or polyvalent carboxylic acid-containing food product >

In the step of allowing the carboxy group-generating enzyme to act on the fatty acid-containing composition or the fatty acid-containing food product, the reaction time, the temperature, the pH of the reaction solution, etc. are not particularly limited. In addition, in the step of allowing the carboxy group-generating enzyme to act on the alcohol-containing composition or the alcohol-containing food product, the reaction time, the temperature, the pH of the reaction solution, etc. are not particularly limited. The reaction temperature is, for example, 10°C to 70°C, preferably 15°C to 50°C, and more preferably 20°C to 40°C. The pH of the reaction solution is, for example, pH 4 to 10, preferably pH 4.5 to 9, and more preferably pH 5 to 8. The reaction time is, for example, 30 seconds to 24 hours, preferably 1 minute to 12 hours, more preferably 10 minutes to 6 hours, or further preferably 30 minutes to 4 hours. Under the above-described reaction conditions, generation of polyvalent carboxylic acid can be more efficiently promoted. These reaction conditions are appropriately selected, depending on the polyvalent carboxylic acid-containing composition of interest. Besides, the optimal reaction conditions may be determined through preliminary experiments.

By using the production method of the present embodiment, a composition or a food product that contains polyvalent carboxylic acid can be produced. One aspect of the method for producing a polyvalent carboxylic acid-containing composition or a polyvalent carboxylic acid-containing food product of the present embodiment includes, for example, the following steps (1) and (2):
(1) a step of preparing a raw material containing fatty acid (a fatty acid-containing composition or a fatty acid-containing food product); and
(2) a step of treating the prepared raw material with a carboxy group-generating enzyme.

Besides, after the step (2), (3) a step of inactivating the enzyme, and/or (4) a step of recovering polyvalent carboxylic acid may be added. In addition, the step (2) may also be a step of treating the raw material in the presence of hydrogen peroxide.

Moreover, one aspect of the method for producing a polyvalent carboxylic acid-containing composition or a polyvalent carboxylic acid-containing food product of the present embodiment includes, for example, the following steps (11) and (12):
(11) a step of preparing a raw material containing alcohol (an alcohol-containing composition or an alcohol-containing food product); and
(12) a step of treating the prepared raw material with a carboxy group-generating enzyme.

Besides, after the step (12), (13) a step of inactivating the enzyme, and/or (14) a step of recovering polyvalent carboxylic acid may be added. In addition, the step (12) may also be a step of treating the raw material in the presence of hydrogen peroxide.

### < Polyvalent carboxylic acid-containing composition/polyvalent carboxylic acid-containing food product >

The present embodiment may relate to a polyvalent carboxylic acid-containing composition produced by the aforementioned method for producing a polyvalent carboxylic acid-containing composition. In addition, the present embodiment may relate to a polyvalent carboxylic acid-containing food product produced by the aforementioned method for producing a polyvalent carboxylic acid-containing food product. The polyvalent carboxylic acid-containing composition or the polyvalent carboxylic acid-containing food product contains polyvalent carboxylic acid, and the polyvalent carboxylic acid is preferably dicarboxylic acid.

The polyvalent carboxylic acid-containing composition or the polyvalent carboxylic acid-containing food product may contain optional components, which are contained in a fatty acid-containing composition or a fatty acid-containing food product. Moreover, the polyvalent carboxylic acid-containing composition or the polyvalent carboxylic acid-containing food product may be in any form, such as powders, a solid, gel, or a liquid (slurry). Of these, the dicarboxylic acid-containing composition is preferably in a liquid (slurry) form.

### (Method for carboxylating fatty acid)

The present embodiment relates to a method for carboxylating fatty acid, including allowing the polypeptide described in any of the above (1) to (3) or the aforementioned carboxy group-generating enzyme agent to act on fatty acid. The fatty acid may be the aforementioned fatty acid, and the preferred range thereof is also the same.

Moreover, since the carboxy group-generating enzyme of the present embodiment also catalyzes a reaction of shortening fatty acid, the carboxy group-generating enzyme may also be a fatty acid-shortening enzyme. That is, another aspect of the present embodiment may relate to a method for shortening fatty acid, including allowing a fatty acid-shortening enzyme agent that contains the polypeptide described in any of the above (1) to (3) to act on fatty acid. In the method for shortening fatty acid, for example, a fatty acid-shortening enzyme is allowed to act on long-chain fatty acid, so that the long-chain fatty acid having a long chain length can be shortened, and so that fatty acid having a shorter carbon chain length than that of the original long-chain fatty acid can be obtained.

As a further aspect, the carboxy group-generating enzyme of the present embodiment catalyzes a reaction of hydroxylating or carboxylating the terminal alkyl group of alkane or fatty acid ester to generate alcohol or carboxylic acid. For this reason, the carboxy group-generating enzyme may be a hydroxyl group-generating enzyme. That is to say, another aspect of the present embodiment relates to a hydroxylation method or a carboxylation method, including allowing a carboxy group-generating enzyme agent (hydroxyl group-generating enzyme agent) that contains the polypeptide described in any of the above (1) to (3), to act on an alkane-containing composition or a fatty acid ester-containing composition.

### (Method for carboxylating alcohol)

The present embodiment relates to a method for carboxylating alcohol, including allowing the polypeptide described in any of the above (1) to (3) or the aforementioned carboxy group-generating enzyme agent to act on alcohol. The alcohol may be the aforementioned alcohol, and the preferred range thereof is also the same.

Moreover, since the carboxy group-generating enzyme of the present embodiment also catalyzes a reaction of shortening long-chain aliphatic alcohol, the carboxy group-generating enzyme may also be a long-chain aliphatic alcohol-shortening enzyme. That is, another aspect of the present embodiment may relate to a method for shortening long-chain aliphatic alcohol, including allowing an enzyme agent for shortening long-chain aliphatic alcohol that contains the polypeptide described in any of the above (1) to (3) to act on long-chain aliphatic alcohol. In the method for shortening long-chain aliphatic alcohol, for example, a long-chain aliphatic alcohol-shortening enzyme is allowed to act on long-chain aliphatic alcohol, so that the long-chain alcohol having a long chain length can be shortened, and so that an alcohol having a shorter carbon chain length than that of the original long-chain aliphatic alcohol can be obtained.

### Examples

Hereinafter, the characteristics of the present invention will be more specifically described in the following examples and comparative examples. The materials, amounts used, proportions, treatment contents, treatment procedures, etc. shown in the following examples may be changed, as appropriate, without deviating from the gist of the present invention. Therefore, the scope of the present invention should not be interpreted limitedly by the specific examples shown below.

### < Enzyme acquisition and identification >

### (Selection of carboxy group-generating enzyme)

Candidates for the carboxy group-generating enzyme were selected from the protein database of NCBI.

### (Preparation of carboxy group-generating enzyme)

Based on the amino acid sequence of each candidate and the codon frequency of *Aspergillus oryzae*, artificial synthetic genes with optimized codons were prepared. A polyhistidine tag was added to the C-terminal side, and the gene was then ligated to a Filamentous fungi expression vector (pUC19, into which a promoter of α-amylase derived from *A. oryzae*, a terminator of glucose dehydrogenase derived from *A. oryzae*, and a pyrG gene had been inserted). The thus created expression vector was introduced into *Aspergillus oryzae* by a protoplast-PEG method to obtain a transformant. The transformant was cultured in liquid at 30°C for 7 days. The resulting culture solution was filtrated and was then purified using TALON Spin Columns (TaKaRa) and HisTALONtm Buffer Set. The crude enzyme solution was concentrated by ultrafiltration, and the pH thereof was then adjusted to a weak base with 0.5 times the amount of a 0.2 M Tris-HCl buffer (pH 8.2). This pH-adjusted solution was applied to a TALON Spin Column equilibrated with Equilibration Buffer. After washing the above-described column with 8 mL of Equilibration Buffer, each of the purified enzymes shown below was obtained by eluting with 1 mL of Elution Buffer.

- Carboxy group-generating enzyme derived from *Marasmius fiardii*
- Carboxy group-generating enzyme derived from *Dendrothele bispora*
- Carboxy group-generating enzyme derived from *Mycena venus*
- Carboxy group-generating enzyme derived from *Armillaria ostoyae*

### (Test Example 1: Carboxylation reaction with each enzyme)

An acetate buffer (pH 5.5) (concentration at reaction: 0.05 mM), a substrate (myristic acid or decanoic acid: concentration at reaction: 100 µM), and each of the purified enzymes described above were added to a 6 cc vial to a final concentration of 0.09 to 20 µM, so that the liquid amount at the reaction become 0.5 mL. Besides, as a control, a reaction solution was also prepared using a known peroxygenase derived from *Marasmius rotula* (UPO derived from *Marasmius rotula*) as an enzyme. Thereafter, hydrogen peroxide was added to each reaction solution to a concentration at the reaction of 10 mM, and the reaction was then carried out at 30°C for 3 hours. It is to be noted that the substrate was dissolved in acetonitrile, and that the final concentration of acetonitrile in the reaction solution was set to be 10% by mass. To 0.5 mL of the reaction solution, 1 mL of chloroform and 5 µL of 6 N HCl were added, and the obtained mixture was then stirred well. Thereafter, 600 µL of the chloroform layer was fractionated into a new vial, and was then air-dried overnight. After the solvent had been distilled away, 300 µL of BSTFA-TMCS (99 : 1) (Tokyo Chemical Industry Co., Ltd.) was added to the vial, and was then left at rest for 1 hour or more. Thereafter, 1 µL of this solution was injected into the GC-MS. The proportion of each product was calculated from the peak area of chromatography.

### GCMS analysis conditions

Column: DB-5
Carrier gas: He
Column flow amount: 2.14 mL
Purge flow amount: 6.0 mL
Split ratio: 16.5
Interface temperature: 200°C
Vaporizer temperature: 250°C
Ion source temperature: 200°C
Column temperature: 50°C (1 min) - 10°C/min (15 min) - 50°C/min (2 min) - 300°C (2 min)

### (Results)

As a result that a reaction was carried out using decanoic acid as a substrate, decanedioic acid was produced in all the enzymes, and the carboxy group-generating activity could be confirmed. In addition, nonanoic acid was produced in some of the enzymes, and it could be confirmed that they also catalyzed a fatty acid-shortening reaction.

**[Table 1]**

| Substrate: decanoic acid (C10) | | | | |
|---|---|---|---|---|
| Candidate enzyme | Content percentage of reaction product | | | |
| | Nonanoic acid (C9) | 9-Hydroxylated decanoic acid (C10) | 10-Hydroxylated decanoic acid (C10) | Decanedioic acid (C10) |
| *Marasmius rotula-derived* UPO | 1.5% | 22.5% | 0.4% | 40.0% |
| *Marasmius fiardii* | 5.7% | 76.3% | 0.8% | 1.1% |
| *Dendrothele bispora* | 16.8% | 7.5% | - | 10.3% |
| *Mycena venus* | - | 10.5% | 0.6% | 0.2% |
| *Armillaria ostoyae* | - | 39.0% | - | 1.0% |

As a result that a reaction was carried out using myristic acid as a substrate, it could be confirmed that dicarboxylic acid was generated in all the enzymes. In some of the enzymes, dicarboxylic acid with a shortened chain length was generated, and it could be confirmed that the enzymes also catalyzed a fatty acid-shortening reaction.

**[Table 2]**

| Substrate: Myristic acid (C14) | | | | |
|---|---|---|---|---|
| Candidate enzyme | Product | | | |
| | Undecanedioic acid (C11) | Dodecanedioic acid (C12) | Tridecanedioic acid (C13) | Tetradecanedioic acid (C14) |
| *Marasmius rotula-derived* UPO | 4.6% | 6.9% | 17.7% | 21.5% |
| *Marasmius fiardii* | 0.2% | 0.2% | 0.2% | 4.4% |
| *Dendrothele bispora* | - | - | 10.0% | 23.0% |
| *Mycena venus* | - | - | - | 13.4% |
| *Armillaria ostoyae* | 7.4% | 7.4% | - | - |

### (Test Example 2: Carboxylation reaction of each substrate using UPO derived from Marasmius rotula)

An acetate buffer (pH 5.5) (concentration at reaction: 0.05 mM), a substrate (lauric acid or dodecanol: concentration at reaction: 100 µM), and each of the purified enzymes described above were added to a 6 cc vial to a final concentration of 0.0001 to 20 µM, so that the liquid amount at the reaction become 0.5 mL. Besides, as a control, a solution was also prepared by subjecting UPO derived from *Marasmius rotula* to heat inactivation. Hydrogen peroxide was added to each reaction solution to a concentration at the reaction of 10 mM, and the reaction was then carried out at 30°C for 3 hours. It is to be noted that the substrate was dissolved in acetonitrile, and that the final concentration of acetonitrile in the reaction solution was set to be 10% by mass. To 0.5 mL of the reaction solution, 1 mL of chloroform and 5 µL of 6 N HCl were added, and the obtained mixture was then stirred well. Thereafter, 600 µL of the chloroform layer was fractionated into a new vial, and was then air-dried overnight. After the solvent had been distilled away, 300 µL of BSTFA-TMCS (99 : 1) (Tokyo Chemical Industry Co., Ltd.) was added to the vial, and was then left at rest for 1 hour or more. Thereafter, 1 µL of this solution was injected into the GC-MS. The proportion of each product was calculated from the peak area of chromatography.

### GCMS analysis conditions

Column: DB-5
Carrier gas: He
Column flow amount: 2.14 mL
Purge flow amount: 6.0 mL
Split ratio: 16.5
Interface temperature: 200°C
Vaporizer temperature: 250°C
Ion source temperature: 200°C
Column temperature: 50°C (1 min) - 10°C/min (15 min) - 50°C/min (2 min) - 300°C (2 min)

### (Results)

As a result that a reaction was carried out using dodecanol as a substrate, consumption of the substrate was confirmed, and a carboxy group-generating activity could be confirmed. In addition, since a reduction in lauric acid by the present enzyme could be confirmed even in the case of using the lauric acid as a substrate, it was assumed that the present enzyme has a dicarboxylic acid-generating ability also to lauric acid.

Qualitative characterization of a product was carried out in the case of using dodecanol as a substrate. As a result, generation of lauric acid and 11-hydroxy lauric acid was confirmed in the case of using the dodecanol as a substrate (Figure 1(a): UPO derived from *Marasmius rotula*; and Figure 1(b): control).

**[Table 3]**

| Enzyme used | Substrate | Substrate-remaining percentage (%) | Product | Amount generated* |
|---|---|---|---|---|
| *Marasmius rotula*-derived UPO | Dodecanol | 0 | Lauric acid | 0.00051105 |
| | | | 11-Hydroxy lauric acid | 0.209497806 |

| | | | | |
|---|---|---|---|---|
| * Amount generated = {(peak area of product when enzyme is used/(peak area of internal standard substance)} - {peak area when heat-inactivated enzyme is used)/(peak area of internal standard substance)} * Internal standard substance: oleic acid Final concentration: 100 µM | | | | |

SEQ ID NO: 1: Mature sequence of chloroperoxidase (Accession No. KAF9265234.1) derived from *Marasmius fiardii* PR-910 
DDEVDFSAHAWQAPGPNDSRSPCPGLNTLANHGFLPRNGKNISIPMIIQAGHDG
YHFEADILTIAAKVGLLTSPDDQPTTLSLNDLKAHGVLEHDASLSRQDFALGDN
VHFNETLFSTLTNSNPGYDVYNITSAGQVQRARLADSVQNNPNITNTDTTLFIRS
AESAFYLSAMGDPIKGVAPKNFVQIFFREERMPIEEGWKRSSTPVTADTLVPLIN
QITAASQPWEPSGKNCPGILP
SEQ ID NO: 2: Mature sequence of SEQ ID NO: 1 + His tag 
DDEVDFSAHAWQAPGPNDSRSPCPGLNTLANHGFLPRNGKNISIPMIIQAGHDG
YHFEADILTIAAKVGLLTSPDDQPTTLSLNDLKAHGVLEHDASLSRQDFALGDN
VHFNETLFSTLTNSNPGYDVYNITSAGQVQRARLADSVQNNPNITNTDTTLFIRS
AESAFYLSAMGDPIKGVAPKNFVQIFFREERMPIEEGWKRSSTPVTADTLVPLIN
QITAASQPWEPSGKNCPGILPHHHHHH
SEQ ID NO: 3: Mature sequence of SEQ ID NO: 1 +signal sequence + His tag 
MKLLSSLVFIAVASTALADDEVDFSAHAWQAPGPNDSRSPCPGLNTLANHGFLP
RNGKNISIPMIIQAGHDGYHFEADILTIAAKVGLLTSPDDQPTTLSLNDLKAHGV
LEHDASLSRQDFALGDNVHFNETLFSTLTNSNPGYDVYNITSAGQVQRARLADS
VQNNPNITNTDTTLFIRSAESAFYLSAMGDPIKGVAPKNFVQIFFREERMPIEEG
WKRSSTPVTADTLVPLINQITAASQPWEPSGKNCPGILPHHHHHH
SEQ ID NO: 4: Mature sequence of chloroperoxidase (Accession No. THU90505.1) derived from *Dendrothele bispora* CBS 962.96 
KNGSAEIDWDAHQWQAPGPNDLRGPCPGLNTLANHGFLPRDGRNLNMSVIVD
AGFDGYHMQPDVLALAVKVAFLTTDDPFSFTLDDIKLHETIEHDASLSREDFALG
DNLHFNETIFSTLANSNPGSDFYNTTSAGEVQRLRLADSEARNPNVTNTDKEFFI
RSAESGFYLSVMGDPLAGVAPKEFVQIFFREERLPIAEGWHRSETPINSTTLGPLI
GKIEAASNWTTSGGCPIIVLGPDFGSF
SEQ ID NO: 5: Mature sequence of SEQ ID NO: 4+ His tag 
KNGSAEIDWDAHQWQAPGPNDLRGPCPGLNTLANHGFLPRDGRNLNMSVIVD
AGFDGYHMQPDVLALAVKVAFLTTDDPFSFTLDDIKLHETIEHDASLSREDFALG
DNLHFNETIFSTLANSNPGSDFYNTTSAGEVQRLRLADSEARNPNVTNTDKEFFI
RSAESGFYLSVMGDPLAGVAPKEFVQIFFREERLPIAEGWHRSETPINSTTLGPLI
GKIEAASNWTTSGGCPIIVLGPDFGSFHHHHHH
SEQ ID NO: 6: Mature sequence of SEQ ID NO: 4+signal sequence + His tag 
MLSKSLLLSFCTTALLASVASAKNGSAEIDWDAHQWQAPGPNDLRGPCPGLNT
LANHGFLPRDGRNLNMSVIVDAGFDGYHMQPDVLALAVKVAFLTTDDPFSFTL
DDIKLHETIEHDASLSREDFALGDNLHFNETIFSTLANSNPGSDFYNTTSAGEVQ
RLRLADSEARNPNVTNTDKEFFIRSAESGFYLSVMGDPLAGVAPKEFVQIFFREE
RLPIAEGWHRSETPINSTTLGPLIGKIEAASNWTTSGGCPIIVLGPDFGSFHHHHH
H
SEQ ID NO: 7: Mature sequence of hypothetical protein (Accession No. KAF7362512.1) derived from *Mycena venus* 
STIDNHQWIAPKATDVRSPCPGLNTLANHGYLPRDGRNISIPMMVQAALDGFNV
GVDAILQAAKFGLLSTDDLMTLNLDALKLHNLIEHDASLSRNDLAVTGDNLHF
NETIFTTLANSNPGVDYYDPTSAGQVQHDRLAISLATNPNITNTAKEFIVRTRESS
FYLSVMGNPLTGVAPKQFVNIFFREERLPIAEGWKKSTTVITGEILNPIMDVIIDN
SNWTMTQLCEDLVLGPNLTF
SEQ ID NO: 8: Mature sequence of SEQ ID NO: 7+ His tag 
STIDNHQWIAPKATDVRSPCPGLNTLANHGYLPRDGRNISIPMMVQAALDGFNV
GVDAILQAAKFGLLSTDDLMTLNLDALKLHNLIEHDASLSRNDLAVTGDNLHF
NETIFTTLANSNPGVDYYDPTSAGQVQHDRLAISLATNPNITNTAKEFIVRTRESS
FYLSVMGNPLTGVAPKQFVNIFFREERLPIAEGWKKSTTVITGEILNPIMDVIIDN
SNWTMTQLCEDLVLGPNLTFHHHHHH
SEQ **ID** NO: 9: Mature sequence of SEQ ID NO: 7+signal sequence + His tag 
MNLLLVSLFLVAVATAKGSTIDNHQWIAPKATDVRSPCPGLNTLANHGYLPRDG
RNISIPMMVQAALDGFNVGVDAILQAAKFGLLSTDDLMTLNLDALKLHNLIEH
DASLSRNDLAVTGDNLHFNETIFTTLANSNPGVDYYDPTSAGQVQHDRLAISLA
TNPNITNTAKEFIVRTRESSFYLSVMGNPLTGVAPKQFVNIFFREERLPIAEGWKK
STTVITGEILNPIMDVIIDNSNWTMTQLCEDLVLGPNLTFHHHHHH
SEQ ID NO: 10: Mature sequence of Chloroperoxidase (Accession No. SJL09533.1) derived from *Armillaria ostoyae* 
TVDWKLHEWQAPGASDLRGPCPGLNTLANHGFLPRDGKNITIKMVLDAGLEG
FNIDPSILILAAKVGLLTTNAPDSFTLDDLKLHGTIEHDASLSRSDFLLGDNLHFN
ETIYTTLTQSNPGVDYFNATSAGQVQKKRLADDIIANPGIINTIKEFTVRTRESAL
YLSVMGDPVTGIAPKKFVDIFFREERMPIEEGWTQPSTLITGQTISPIADIIEETSE
WVDNGGCPIIRPGPNSDLISCP
SEQ ID NO: 11: Mature sequence of SEQ ID NO: 10+ His tag 
TVDWKLHEWQAPGASDLRGPCPGLNTLANHGFLPRDGKNITIKMVLDAGLEG
FNIDPSILILAAKVGLLTTNAPDSFTLDDLKLHGTIEHDASLSRSDFLLGDNLHFN
ETIYTTLTQSNPGVDYFNATSAGQVQKKRLADDIIANPGIINTIKEFTVRTRESAL
YLSVMGDPVTGIAPKKFVDIFFREERMPIEEGWTQPSTLITGQTISPIADIIEETSE
WVDNGGCPIIRPGPNSDLISCPHHHHHH
SEQ ID NO: 12: Mature sequence of SEQ ID NO: 10+signal sequence + His tag 
MYLSLTFVAAFFSAVHATVDWKLHEWQAPGASDLRGPCPGLNTLANHGFLPRD
GKNITIKMVLDAGLEGFNIDPSILILAAKVGLLTTNAPDSFTLDDLKLHGTIEHDA
SLSRSDFLLGDNLHFNETIYTTLTQSNPGVDYFNATSAGQVQKKRLADDIIANPG
IINTIKEFTVRTRESALYLSVMGDPVTGIAPKKFVDIFFREERMPIEEGWTQPSTLI
TGQTISPIADIIEETSEWVDNGGCPIIRPGPNSDLISCPHHHHHH
SEQ ID NO: 13: Nucleotide sequence (cDNA sequence) encoding mature sequence of Chloroperoxidase (Accession No. KAF9265234.1) derived from *Marasmius fiardii* PR-910 
gatgatgaggtggatttcagtgctcacgcttggcaagctccaggcccgaacgatagtcgatccccatgtcctggccttaacaca
ctcgccaatcatggctttctaccacgcaatggcaagaatatctcgattcccatgatcatccaggcaggacatgacgggtatcact
tcgaggcagatatcctgactatcgctgcgaaagttgggctcttgacgagtccagacgatcaacctaccacattgtcccttaacg
acctgaaagcccatggtgtactggagcatgacgcttcgctaagcagacaggactttgcattaggcgacaacgtgcacttcaac
 
gagactctcttttccacgctgaccaatagcaatccaggttatgacgtgtacaacatcacctcagcaggtcaagtgcaacgtgcc
aggcttgcggatagcgtccagaacaatccgaacattaccaatacagacacgacgctcttcattcgctctgccgaatcggcgtttt
acctgtctgcgatgggagatccgataaagggtgtagcacccaagaacttcgtccagatcttctttcgggaagagcgtatgccg
attgaggaagggtggaaacggagctcaacccctgttactgccgatacacttgtccccttgatcaaccagataaccgctgcatct
cagccctgggaaccctctggaaagaattgccctggaattcttcct
SEQ ID NO: 14: Nucleotide sequence encoding mature sequence of SEQ ID NO: 13 + His tag 
gatgatgaggtggatttcagtgctcacgcttggcaagctccaggcccgaacgatagtcgatccccatgtcctggccttaacaca
ctcgccaatcatggctttctaccacgcaatggcaagaatatctcgattcccatgatcatccaggcaggacatgacgggtatcact
tcgaggcagatatcctgactatcgctgcgaaagttgggctcttgacgagtccagacgatcaacctaccacattgtcccttaacg
acctgaaagcccatggtgtactggagcatgacgcttcgctaagcagacaggactttgcattaggcgacaacgtgcacttcaac
gagactctcttttccacgctgaccaatagcaatccaggttatgacgtgtacaacatcacctcagcaggtcaagtgcaacgtgcc
aggcttgcggatagcgtccagaacaatccgaacattaccaatacagacacgacgctcttcattcgctctgccgaatcggcgtttt
acctgtctgcgatgggagatccgataaagggtgtagcacccaagaacttcgtccagatcttctttcgggaagagcgtatgccg
attgaggaagggtggaaacggagctcaacccctgttactgccgatacacttgtccccttgatcaaccagataaccgctgcatct
cagccctgggaaccctctggaaagaattgccctggaattcttcctcaccatcaccatcatcattag
SEQ ID NO: 15: Nucleotide sequence encoding mature sequence of SEQ ID NO: 13 + signal sequence + His tag 
atgaagctcttgtcatcgctagtcttcattgccgttgcttccactgcgttagccgatgatgaggtggatttcagtgctcacgcttgg
caagctccaggcccgaacgatagtcgatccccatgtcctggccttaacacactcgccaatcatggctttctaccacgcaatggc
aagaatatctcgattcccatgatcatccaggcaggacatgacgggtatcacttcgaggcagatatcctgactatcgctgcgaaa
gttgggctcttgacgagtccagacgatcaacctaccacattgtcccttaacgacctgaaagcccatggtgtactggagcatgac
gcttcgctaagcagacaggactttgcattaggcgacaacgtgcacttcaacgagactctcttttccacgctgaccaatagcaatc
caggttatgacgtgtacaacatcacctcagcaggtcaagtgcaacgtgccaggcttgcggatagcgtccagaacaatccgaa
cattaccaatacagacacgacgctcttcattcgctctgccgaatcggcgttttacctgtctgcgatgggagatccgataaagggt
gtagcacccaagaacttcgtccagatcttctttcgggaagagcgtatgccgattgaggaagggtggaaacggagctcaaccc
ctgttactgccgatacacttgtccccttgatcaaccagataaccgctgcatctcagccctgggaaccctctggaaagaattgccc
tggaattcttcctcaccatcaccatcatcattag
SEQ ID NO: 16: Nucleotide sequence (cDNA sequence) encoding mature sequence of Chloroperoxidase (Accession No. THU90505.1) derived from *Dendrothele bispora* CBS 962.96 
aagaatggctcggcagagattgattgggatgcacatcagtggcaagcacctggacccaatgacctgagaggaccttgtccag
ggctcaacacgttggctaatcacggtttccttcctcgtgatggtcgaaacctcaatatgtcggtgattgtggatgccggatttgac
 
ggctatcacatgcaaccggatgtactcgctttggcggtcaaagtcgcgttcttgaccactgatgaccccttttccttcacactcga
cgatatcaagctccatgagacgatcgagcatgacgcttctctgtcacgggaagactttgcgctaggggataaccttcacttcaa
cgaaaccatcttctctacactcgccaacagcaatccgggttccgacttctacaacacgacttcagcaggcgaagtacagcgttt
acgccttgcggatagtgaagcccgaaacccgaatgtgacgaacacagacaaagagttctttattcgcagtgcggaatccggct
tctacctgtcggttatgggcgatccacttgccggagttgctccaaaggagttcgtccagatcttctttcgcgaggagaggcttcc
catagctgagggttggcatcggtcagaaacccccataaacagcaccacactgggaccactcattgggaagattgaagcagcc
agcaattggaccacttctggtggttgcccgatcatcgttttggggcctgactttggcagtttt
SEQ ID NO: 17: Nucleotide sequence encoding mature sequence of SEQ ID NO: 16 + His tag 
aagaatggctcggcagagattgattgggatgcacatcagtggcaagcacctggacccaatgacctgagaggaccttgtccag
ggctcaacacgttggctaatcacggtttccttcctcgtgatggtcgaaacctcaatatgtcggtgattgtggatgccggatttgac
ggctatcacatgcaaccggatgtactcgctttggcggtcaaagtcgcgttcttgaccactgatgaccccttttccttcacactcga
cgatatcaagctccatgagacgatcgagcatgacgcttctctgtcacgggaagactttgcgctaggggataaccttcacttcaa
cgaaaccatcttctctacactcgccaacagcaatccgggttccgacttctacaacacgacttcagcaggcgaagtacagcgttt
acgccttgcggatagtgaagcccgaaacccgaatgtgacgaacacagacaaagagttctttattcgcagtgcggaatccggct
tctacctgtcggttatgggcgatccacttgccggagttgctccaaaggagttcgtccagatcttctttcgcgaggagaggcttcc
catagctgagggttggcatcggtcagaaacccccataaacagcaccacactgggaccactcattgggaagattgaagcagcc
agcaattggaccacttctggtggttgcccgatcatcgttttggggcctgactttggcagttttcaccatcaccatcatcattag
SEQ ID NO: 18: Nucleotide sequence encoding mature sequence of SEQ ID NO: 16 + signal sequence + His tag 
atgctgtcgaaatccctgctactatccttctgcaccactgccttgttagcctctgtcgctagcgctaagaatggctcggcagagat
tgattgggatgcacatcagtggcaagcacctggacccaatgacctgagaggaccttgtccagggctcaacacgttggctaatc
acggtttccttcctcgtgatggtcgaaacctcaatatgtcggtgattgtggatgccggatttgacggctatcacatgcaaccggat
gtactcgctttggcggtcaaagtcgcgttcttgaccactgatgaccccttttccttcacactcgacgatatcaagctccatgagac
gatcgagcatgacgcttctctgtcacgggaagactttgcgctaggggataaccttcacttcaacgaaaccatcttctctacactc
gccaacagcaatccgggttccgacttctacaacacgacttcagcaggcgaagtacagcgtttacgccttgcggatagtgaagc
ccgaaacccgaatgtgacgaacacagacaaagagttctttattcgcagtgcggaatccggcttctacctgtcggttatgggcga
tccacttgccggagttgctccaaaggagttcgtccagatcttctttcgcgaggagaggcttcccatagctgagggttggcatcg
gtcagaaacccccataaacagcaccacactgggaccactcattgggaagattgaagcagccagcaattggaccacttctggt
ggttgcccgatcatcgttttggggcctgactttggcagttttcaccatcaccatcatcattag
SEQ ID NO: 19: Nucleotide sequence (cDNA sequence) encoding mature sequence of hypothetical protein (Accession No. KAF7362512.1) derived from *Mycena venus* 
tctacgattgacaatcaccagtggattgcacctaaagcgacggatgtccggtcaccatgtcctgggctgaatacacttgcaaac
catggctatctgcctagggatggccgcaacatcagtataccgatgatggtccaagctgccttagacgggttcaatgtaggtgtc
gatgcgattctacaagccgctaagtttggccttctaagcactgatgatctcatgacccttaacttggatgccttgaagttacacaac
ctgatcgagcatgatgccagtttgtcccgtaacgacttggcagtgacaggagacaaccttcacttcaacgagacaatcttcacg
acacttgcgaattccaatccaggtgttgactactatgaccccacttcagctggacaagtgcagcatgatcgactcgcaattagct
tggcaaccaatcccaacataaccaataccgccaaggagttcatcgtcagaacgcgggaatcttcgttctacctctcggttatgg
gcaatccgcttactggtgttgctccgaaacagttcgtgaacatcttctttcgcgaagaacgtctgccaattgctgaagggtggaa
gaagagcactaccgtcattacgggagagatcctgaatcccataatggacgtcatcatcgacaactctaactggaccatgactca
gctatgcgaggatctcgtgctcggtcctaatctcaccttt
SEQ ID NO: 20: Nucleotide sequence encoding mature sequence of SEQ ID NO: 19 + His tag 
tctacgattgacaatcaccagtggattgcacctaaagcgacggatgtccggtcaccatgtcctgggctgaatacacttgcaaac
catggctatctgcctagggatggccgcaacatcagtataccgatgatggtccaagctgccttagacgggttcaatgtaggtgtc
gatgcgattctacaagccgctaagtttggccttctaagcactgatgatctcatgacccttaacttggatgccttgaagttacacaac
ctgatcgagcatgatgccagtttgtcccgtaacgacttggcagtgacaggagacaaccttcacttcaacgagacaatcttcacg
acacttgcgaattccaatccaggtgttgactactatgaccccacttcagctggacaagtgcagcatgatcgactcgcaattagct
tggcaaccaatcccaacataaccaataccgccaaggagttcatcgtcagaacgcgggaatcttcgttctacctctcggttatgg
gcaatccgcttactggtgttgctccgaaacagttcgtgaacatcttctttcgcgaagaacgtctgccaattgctgaagggtggaa
gaagagcactaccgtcattacgggagagatcctgaatcccataatggacgtcatcatcgacaactctaactggaccatgactca
gctatgcgaggatctcgtgctcggtcctaatctcacctttcaccatcaccatcatcattag
SEQ ID NO: 21: Nucleotide sequence encoding mature sequence of SEQ ID NO: 19 + signal sequence + His tag 
atgaacctgctcttggtgtccctatttctggtagctgttgccacagcgaaaggctctacgattgacaatcaccagtggattgcacc
taaagcgacggatgtccggtcaccatgtcctgggctgaatacacttgcaaaccatggctatctgcctagggatggccgcaaca
tcagtataccgatgatggtccaagctgccttagacgggttcaatgtaggtgtcgatgcgattctacaagccgctaagtttggcctt
ctaagcactgatgatctcatgacccttaacttggatgccttgaagttacacaacctgatcgagcatgatgccagtttgtcccgtaa
cgacttggcagtgacaggagacaaccttcacttcaacgagacaatcttcacgacacttgcgaattccaatccaggtgttgacta
ctatgaccccacttcagctggacaagtgcagcatgatcgactcgcaattagcttggcaaccaatcccaacataaccaataccgc
caaggagttcatcgtcagaacgcgggaatcttcgttctacctctcggttatgggcaatccgcttactggtgttgctccgaaacagt
tcgtgaacatcttctttcgcgaagaacgtctgccaattgctgaagggtggaagaagagcactaccgtcattacgggagagatcc
tgaatcccataatggacgtcatcatcgacaactctaactggaccatgactcagctatgcgaggatctcgtgctcggtcctaatct
cacctttcaccatcaccatcatcattag
SEQ ID NO: 22: Nucleotide sequence (cDNA sequence) encoding mature sequence of Chloroperoxidase (Accession No. SJL09533.1) derived from *Armillaria ostoyae* 
actgtcgattggaaacttcacgaatggcaagctccaggagcatcggacttacgtggtccttgtccgggattgaacactttggcc
aatcacggctttctacccagagacggaaagaacattacgatcaagatggtgcttgatgccggtctagagggattcaacatagat
ccctccatcctgattttggctgccaaagtcggcttactcaccacaaatgcaccagacagctttacgcttgatgacctgaagctgc
atgggaccattgagcatgatgcgtctttgtcacgctctgatttcctcctcggtgataacttgcacttcaatgagacgatctacacca
cccttacgcagtccaatcctggtgtggactacttcaacgcgacatctgctggccaagtccagaaaaaacggcttgcggatgac
atcattgccaacccagggatcatcaacaccattaaggagttcacagttcgaacgcgggaaagtgccctctatctctccgtaatg
ggcgatccagttactggcattgctcccaagaagttcgtggacatattctttcgcgaagagaggatgcctattgaagagggatgg
actcaaccgagtaccctaatcacagggcagacaatctcgcctatagctgacatcatcgaggaaacttcggaatgggtcgataa
tggtggctgcccgattatccgtcctggtccgaatagcgacctgatcagctgcccc
SEQ ID NO: 23: Nucleotide sequence encoding mature sequence of SEQ ID NO: 22 + His tag 
actgtcgattggaaacttcacgaatggcaagctccaggagcatcggacttacgtggtccttgtccgggattgaacactttggcc
aatcacggctttctacccagagacggaaagaacattacgatcaagatggtgcttgatgccggtctagagggattcaacatagat
ccctccatcctgattttggctgccaaagtcggcttactcaccacaaatgcaccagacagctttacgcttgatgacctgaagctgc
atgggaccattgagcatgatgcgtctttgtcacgctctgatttcctcctcggtgataacttgcacttcaatgagacgatctacacca
cccttacgcagtccaatcctggtgtggactacttcaacgcgacatctgctggccaagtccagaaaaaacggcttgcggatgac
atcattgccaacccagggatcatcaacaccattaaggagttcacagttcgaacgcgggaaagtgccctctatctctccgtaatg
ggcgatccagttactggcattgctcccaagaagttcgtggacatattctttcgcgaagagaggatgcctattgaagagggatgg
actcaaccgagtaccctaatcacagggcagacaatctcgcctatagctgacatcatcgaggaaacttcggaatgggtcgataa
tggtggctgcccgattatccgtcctggtccgaatagcgacctgatcagctgcccccaccatcaccatcatcattag
SEQ ID NO: 24: Nucleotide sequence encoding mature sequence of SEQ ID NO: 22 + signal sequence + His tag

atgtatctctcactgacctttgtagcagccttcttttccgcggttcatgcaactgtcgattggaaacttcacgaatggcaagctcca
ggagcatcggacttacgtggtccttgtccgggattgaacactttggccaatcacggctttctacccagagacggaaagaacatt
acgatcaagatggtgcttgatgccggtctagagggattcaacatagatccctccatcctgattttggctgccaaagtcggcttact
caccacaaatgcaccagacagctttacgcttgatgacctgaagctgcatgggaccattgagcatgatgcgtctttgtcacgctct
gatttcctcctcggtgataacttgcacttcaatgagacgatctacaccacccttacgcagtccaatcctggtgtggactacttcaa
cgcgacatctgctggccaagtccagaaaaaacggcttgcggatgacatcattgccaacccagggatcatcaacaccattaag
gagttcacagttcgaacgcgggaaagtgccctctatctctccgtaatgggcgatccagttactggcattgctcccaagaagttc
gtggacatattctttcgcgaagagaggatgcctattgaagagggatggactcaaccgagtaccctaatcacagggcagacaat
ctcgcctatagctgacatcatcgaggaaacttcggaatgggtcgataatggtggctgcccgattatccgtcctggtccgaatag
cgacctgatcagctgcccccaccatcaccatcatcattag

## Claims

1. A carboxy group-generating enzyme agent, containing a polypeptide described in any of the following (1) to (3):
(1) a polypeptide consisting of the amino acid sequence as set forth in any of the amino acid sequence as set forth in any of SEQ ID NOs: 1, 2, 4, 5, 7, 8, 10 and 11;
(2) a polypeptide having a substitution, an addition, an insertion and/or a deletion of one or several amino acid residues in the amino acid sequence as set forth in any of SEQ ID NOs: 1, 2, 4, 5, 7, 8, 10 and 11, and having a carboxy group-generating activity; and
(3) a polypeptide having a sequence identity of 90% or more to the amino acid sequence as set forth in any of SEQ ID NOs: 1, 2, 4, 5, 7, 8, 10 and 11, and having a carboxy group-generating activity.

2. The carboxy group-generating enzyme agent according to claim 1, which is for use in dicarboxylic acid generation.

3. A method for producing a polyvalent carboxylic acid-containing composition, including allowing the polypeptide or the carboxy group-generating enzyme agent according to claim 1 or 2 to act on a fatty acid-containing composition.

4. The method for producing a polyvalent carboxylic acid-containing composition according to claim 3, in which the fatty acid-containing composition contains decanoic acid or myristic acid.

5. The method for producing a polyvalent carboxylic acid-containing composition according to claim 3, in which the polyvalent carboxylic acid-containing composition is a dicarboxylic acid-containing composition.

6. A method for carboxylating fatty acid, including allowing the polypeptide or the carboxy group-generating enzyme agent according to claim 1 or 2 to act on fatty acid.

7. A method for producing a polyvalent carboxylic acid-containing food product, including allowing the polypeptide or the carboxy group-generating enzyme agent according to claim 1 or 2 to act on a fatty acid-containing food product.

8. The method for producing a polyvalent carboxylic acid-containing food product according to claim 7, in which the polyvalent carboxylic acid-containing food product is a dicarboxylic acid-containing food product.
